(19) **Europäisches Patentamt / European Patent Office / Office européen des brevets**

(11) **EP 2 198 900 B1**

(12) **EUROPEAN PATENT SPECIFICATION**

(45) Date of publication and mention
of the grant of the patent:
**10.02.2016 Bulletin 2016/06**

(51) Int Cl.:
***A61M 1/36*** *(2006.01)*

(21) Application number: **09165573.8**

(22) Date of filing: **22.10.1999**

(54) **Method and device for detecting access recirculation**

Verfahren und Vorrichtung zum Erkennen einer Rezirkulation

Procédé et dispositif de détection de recirculation d'accès

(84) Designated Contracting States:
**AT BE CH CY DE DK ES FI FR GB GR IE IT LI LU MC NL PT SE**

(30) Priority: **23.10.1998 US 105396 P**

(43) Date of publication of application:
**23.06.2010 Bulletin 2010/25**

(62) Document number(s) of the earlier application(s) in accordance with Art. 76 EPC:
**99970926.4 / 1 124 599**

(73) Proprietor: **Gambro Lundia AB**
**220 10 Lund (SE)**

(72) Inventor: **Sternby, Jan**
**SE-226 62, Lund (SE)**

(74) Representative: **Bornegard, Annette**
**Gambro AB**
**P.O. Box 10101**
**S-220 10 Lund (SE)**

(56) References cited:
**EP-A1- 0 693 296      DE-C1- 19 528 907**
**DE-C1- 19 541 783      DE-C1- 19 702 441**

## Description

AREA OF INVENTION

The present invention relates to a method and device for measuring blood flow rate in a blood access. Blood is taken out from the body of a mammal to an extracorporeal blood circuit through a blood access, via needles or a catheter.

PRIOR ART

There are several types of treatments in which blood is taken out in an extracorporeal blood circuit Such treatments involve, for example, hemodialysis, hemofiltration, hemodiafiltration, plasmapheresis, blood component separation, blood oxygenation, etc. Normally, blood is removed from a blood vessel at an access site and returned to the same blood vessel or at another location in the body.

In hemodialysis and similar treatments, an access site is commonly surgically created in the nature of a fistula. Blood needles are inserted in the area of the fistula.. Blood is taken out from the fistula via an arterial needle and blood is returned to the fistula via a venous needle

A common method of generating a permanent access site having capability of providing a high blood flow and being operative during several years and even tens of years, is the provision of an arterio-venous fistula. It is produced by operatively connecting the radial artery to the cephalic vein at the level of the forearm. The venous limb of the fistula thickens during the course of several months, permitting repeated insertion of dialysis needles.

An alternative to the arterio-venous fistula is the arterio-venous graft, in which a connection is generated from, for example, the radial artery at the wrist to the basilic vein. The connection is made with a tube graft made from autogenous saphenous vein or from polytetrafluorethylene (PTFE, Teflon). The needles are inserted in the graft.

A third method for blood access is to use a silicon, dual-lumen catheter surgically implanted into one of the large veins.

Further methods find use in specific situations, like a no-needle arterio-venous graft consisting of a T-tube linked to a standard PTFE graft. The T-tube is implanted in the skin. Vascular access is obtained either by unscrewing a plastic plug or by puncturing a septum of said T-tube with a needle. Other methods are also known.

During hemodialysis, it is desirable to obtain a constant blood flow rate of 150 - 500 ml/min or even higher, and the access site must be prepared for delivering such flow rates. The blood flow in an AV fistula is often 800 ml/min or larger, permitting delivery of a blood flow rate in the desired range..

In the absence of a sufficient forward blood flow, the extracorporeal circuit blood pump will take up some of the already treated blood entering the fistula via the venous needle, so called access or fistula recirculation, leading to poor treatment results.

The most common cause of poor flow with AV fistulas is partial obstruction of the venous limb due to fibrosis secondary to multiple venipunctures. Moreover, stenosis causes a reduction of access flow.

When there is a problem with access flow, it has been found that access flow rate often exhibit a long plateau time period with reduced but sufficient access flow, followed by a short period of a few weeks with markedly reduced access flow leading to recirculation and ultimately access failure. By constantly monitoring the evolution of the access flow during consecutive treatment sessions, it is possible to detect imminent access flow problems.

Several methods have been suggested for monitoring recirculation and access flow. Many of these methods involve injection of a marker substance in blood, and the resultant recirculation is detected. The methods normally involve measurement of a property in the extracorporeal blood circuit. Examples of such methods can be found in US 5,685,989, US 5,595,182, US 5,453,576, US 5,510,716, US 5, 510, 717, US 5,312,550, etc.

Such methods have the disadvantage that they cannot detect when the access flow has decreased to such an extent that recirculation is at risk, but only when recirculation prevails. Moreover, it is a drawback that injection of a substance is necessary.

A noninvasive technique that allows imaging of flow through AV grafts is color Doppler ultrasound. However, this technique requires expensive equipment.

The measurement of access flow rate necessitates the reversal of the flows in the extracorporeal circuit. A valve for such reversal is shown in i.a. US 5605630 and US 5894011. However, these valve constructions comprises dead ends in which blood may stand still for a long time and coagulate, which is a drawback.

DE 195 41 783 C1 is directed to a method for operating a hemotherapeutic device for determining hemodynamic parameters during an extracorporeal hemotherapy, as well as to a device for determining hemodynamic parameters during an extracorporeal hemotherapy. The fistula flow QF and/or the body temperature TB and/or the cardiac output (l/min) CO are determined.

DE 197 02 441 C1 is directed to a method and a device for determining recirculation during an extracorporeal hemotherapy by providing a short concentration bolus in the dialysis fluid. After the time of the recirculation a portion of the blood is reappearing in the dialyzer and cause again a change of the concentration of the dialysis fluid.

DISCLOSURE OF INVENTION

An apparatus for detecting access recirculation and a method of controlling such an apparatus are provided in accordance with the independent and dependent claims.

It is possible to discriminate between the condition when access or fistula recirculation has developed and not. A method for that purpose would be: changing the blood flow rate (Qb); monitoring the concentration of said substance in the dialysate emitted from the dialyzer; and detecting a possible fistula recirculation in the normal position by correlating a change in said concentration to said change of the blood flow rate..

Preferably, the blood flow rate is decreased and a corresponding decrease in the urea concentration is monitored, and the abscence of such a decrease being indicative of fistula recirculation.

SHORT DESCRIPTION OF DRAWINGS

Further objects, advantages and features of the invention appears from the following detailed description of the invention with reference to specific embodiments of the invention shown on the drawings, in which

Fig. 1 is a partially schematic view of a forearm of a patient provided with an AV fistula
Fig. 2 is a schematic diagram of an extracorporeal dialysis circuit
Fig. 3 is a schematic diagram of the blood flow circuit in a patient and in the attached extracorporeal blood circuit.
Fig. 4 is a schematic diagram similar to Fig. 3, but with the extracorporeal circuit in an alternative reversed position.
Fig. 5 is a schematic diagram of a blood flow circuit including a switch valve.
Fig. 6 is a diagram of the dialysis fluid urea concentration versus time, including a portion with reversed flow access according to the invention..
Fig. 7 is a schematic diagram similar to the diagram of Fig. 5 comprising an alternative valve arrangement.
Fig. 8 is schematic diagram similar to the diagram of Fig.. 7 showing the valve arrangement in an idle postion..
Fig. 9 is schematic diagram similar to the diagram of Fig. 7 showing the valve arrangement in a reversed postion.
Fig. 10 is a schematic diagram similar to Fig.. 5 with the pump in an alternative position..
Fig. 11 is a diagram showing calculations with relative whole body efficiency.
Fig. 12 is a cross-sectional view of a valve housing to be used in the schematic diagram of Figs. 5 and 7 to 10.
Figl 13 is a bottom view of a valve member intended to be inserted in the valve housing of Fig. 12..
Fig. 14 is a partially schematic plan view of the valve housing of Fig. 12.

DESCRIPTION OF DETAILED EMBODIMENTS OF THE INVENTION

For the purpose of this description, an access site is a site in which a fluid in a tube can be accessed and removed from and/or returned to the tube. The tube may be a blood vessel of a mammal, or any other tube in which a fluid is flowing. The access flow rate is the flow rate of the fluid in the tube or blood vessel immediately upstream of the access site or removal position

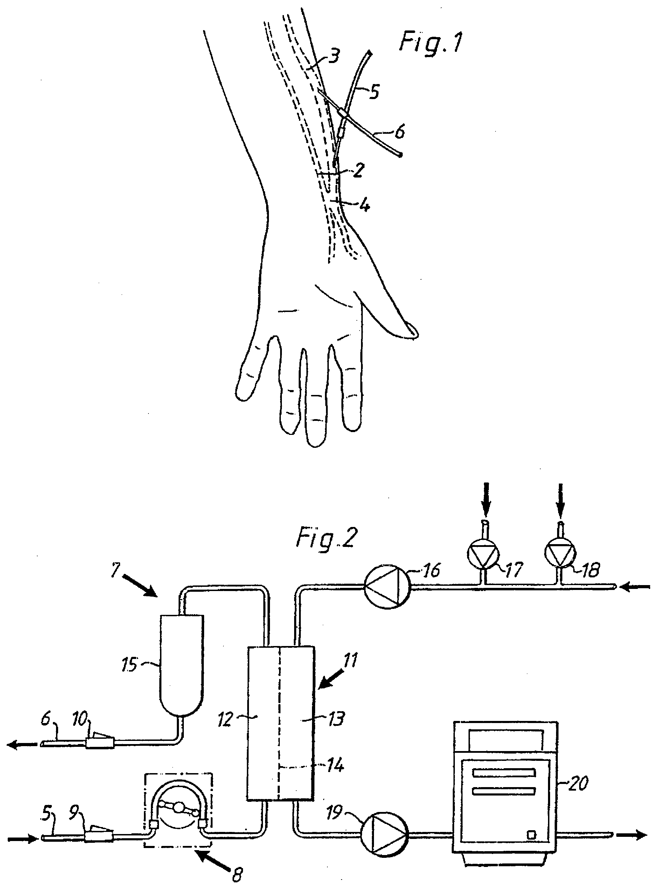

Fig. 1 discloses a forearm 1 of a human patient. The forearm 1 comprises an artery 2, in this case the radial artery, and a vein 3, in this case the cephalic vein. Openings are surgically created in the artery 2 and the vein 3 and the openings are connected to form a fistula 4, in which the arterial blood flow is cross-circuited to the vein. Due to the fistula, the blood flow through the artery and vein is increased and the vein forms an thickened area downstream of the connecting openings. When the fistula has matured after a few months, the vein is thicker and may be punctured repeatedly. Normally, the thickened vein area is called a fistula.

An arterial needle 5 is placed in the fistula, in the enlarged vein close to the connected openings and a venous needle 6 is placed downstream of the arterial needle, normally at least five centimeters downstream thereof..

The needles 5 and 6 are connected to a tube system 7, shown in Fig. 2, forming an extracorporeal circuit comprising a blood pump 8, such as a dialysis circuit. The blood pump propels blood from the blood vessel, through the arterial needle, the extracorporeal circuit, the venous needle and back into the blood vessel.

The extracorporeal blood circuit 7 shown in Fig. 2 further comprises an arterial clamp 9 and a venous clamp 10 for isolating the patient from the extracorporeal circuit should an error occur.

Downstream of pump 8 is a dialyzer 11, comprising a blood compartment 12 and a dialysis fluid compartment 13 separated by a semipermeable membrane 14. Further downstream of the dialyzer is a drip chamber 15, separating air from the blood therein.

Blood passes from the arterial needle past the arterial clamp 9 to the blood pump 8. The blood pump drivs the blood through the dialyzer 11 and further via the drip chamber 15 and past the venous clamp 10 back to the patient via the venous needle. The drip chamber may comprise an air detector, adapted to trigger an alarm should the blood emitted from the drip chamber comprise air or air bubbles. The blood circuit may comprise further components, such as pressure sensors etc

The dialysis fluid compartment 14 of the dialyzer 1.1 is provided with dialysis fluid via a first pump 16, which obtains dialysis fluid from a source of pure water, normally RO-water, and one or several concentrates of ions, metering pumps 17 and 18 being shown for metering such concentrates. The preparation of dialysis fluid is conventional and is not further described here.

An exchange of substances between the blood and the dialysis fluid takes place in the dialyzer through the semipermeable membrane. Notably, urea is passed from the blood, through the semipermeable membrane and to the dialysis fluid present at the other side of the membrane.. The exchange may take place by diffusion under the influence of a concentration gradient, so called hemodialysis, and/or by convection due to a flow of liquid from the blood to the dialysis fluid, so called ultrafiltration, which is an important feature of hemodiafiltration or hemofiltration.

From the dialysis fluid compartment 14 of the dialyzer is emitted a fluid called the dialysate, which is driven by a second pump 19 via a urea monitor 20 to drain. The urea monitor continuously measures the urea concentration in the dialysate emitted from the dialyzer, to provide a dialysate urea, concentration curve during a dialysis treatment. Such urea concentration curve may be used for several purposes, such as obtaining a total body urea mass, as described in WO 9855166, and to obtain a prediction of the whole body dialysis dose Kt/V as also described in said application.

As described above, the present invention provides a method of non-invasively measuring the access flow in the fistula immediately before the arterial needle, using the urea monitor and the dialysis circuit as shown in Fig. 2.

By measuring the dialysis urea concentration during normal dialysis and then reversing the positions of the needles and measuring the dialysis urea concentration with the needles in the reversed position, it is possible to calculate the blood flow in the blood access, without the addition of any substance to blood or the dialysis fluid.

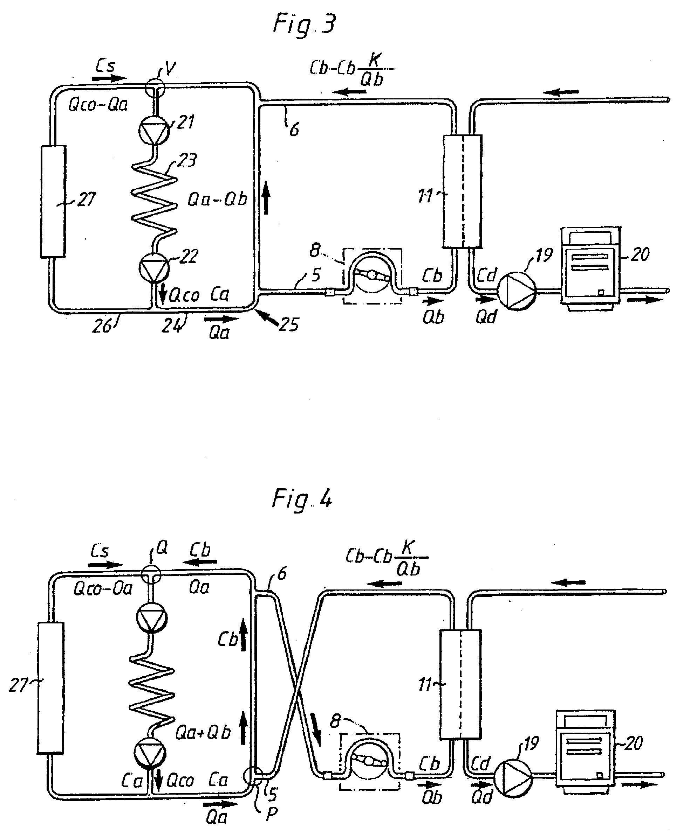

Fig. 3 shows a simplified schematic diagram of the blood vessel circuit of a patient and a portion of the dialysis circuit according to Fig. 2. The patient blood circuit comprises the heart, where the right chamber of the heart is symbolized by an upper pump 21 and the left chamber of the heart is symbolized by a lower pump 22. The lungs 23 are located between the upper and lower pump. From the outlet of the left chamber pump 22 of the heart, the blood flow divides into a first branch 24 leading to the access 25, normally in the left forearm of the patient, and a second branch 26 leading to the rest of the body, such as organs, other limbs, head, etc. symbolized by a block 27. Blood returning from the body from the organs etc., i.e. from block 27, combines with blood returning from the access and enters the right chamber pump 21.

The cardiac output flow rate is defined as Qco and the flow rate of the access is defined as Qa, which means that Qco - Qa enters the block 27. The venous blood returning from block 27 before being mixed with blood from the access, the systemic venous blood, has a urea concentration of Cs. The blood leaving the left chamber pump 22 has a urea concentration of Ca equal to that passing out to the access 25 as well as to the block 27.

For measuring the access flow rate, it is necessary to reverse the flow through the arterial and venous needles. One way of achieving that is to reverse the needles manually.

Alternatively, Fig.. 5 shows a valve 28 for performing the same operation. The arterial needle 5 is connected to an arterial inlet line 29 of the valve and the venous needle 6 is connected to a venous inlet line 30 of the valve. The blood pump is connected to a first outlet line 31. of the valve and the returning blood from the dialyzer 11 is connected to a second outlet line 32 of the valve..

The valve comprises a valve housing and a pivotable valve member 33, which is pivotable from the normal position shown on the drawing to a reverse position pivoted 90° in relation to the normal position.

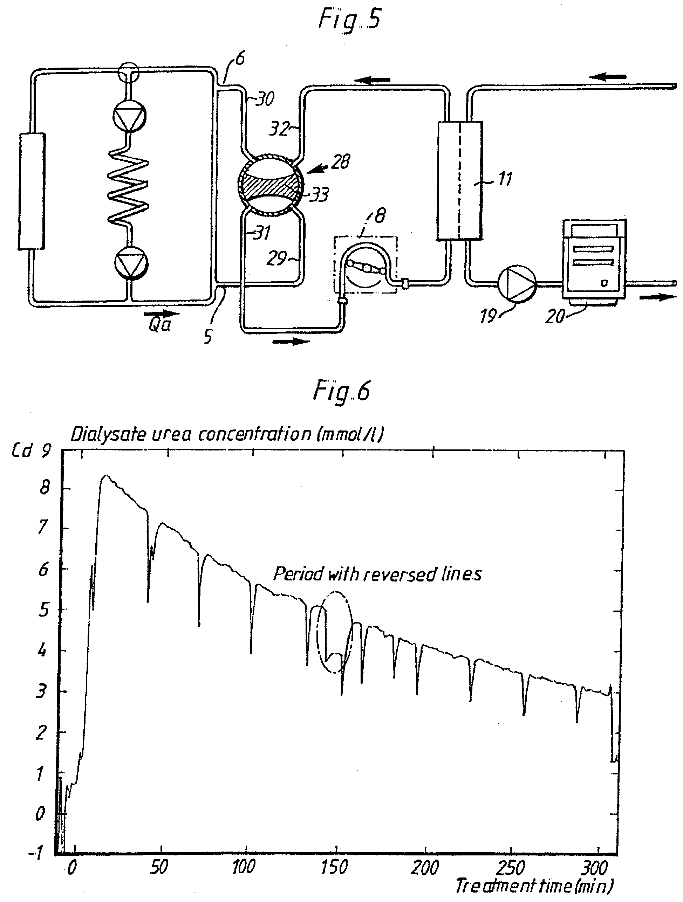

In the normal position shown in Fig. 5, the arterial needle 5 is connected to the blood pump 8 and the venous needle 6 is connected to the outlet of the dialyzer, via the drip chamber, see Fig. 2. In the reversed position, the arterial needle 5 is connected to the outlet of the dialyzer and the venous needle 6 is connected to the blood pump 8, as required.

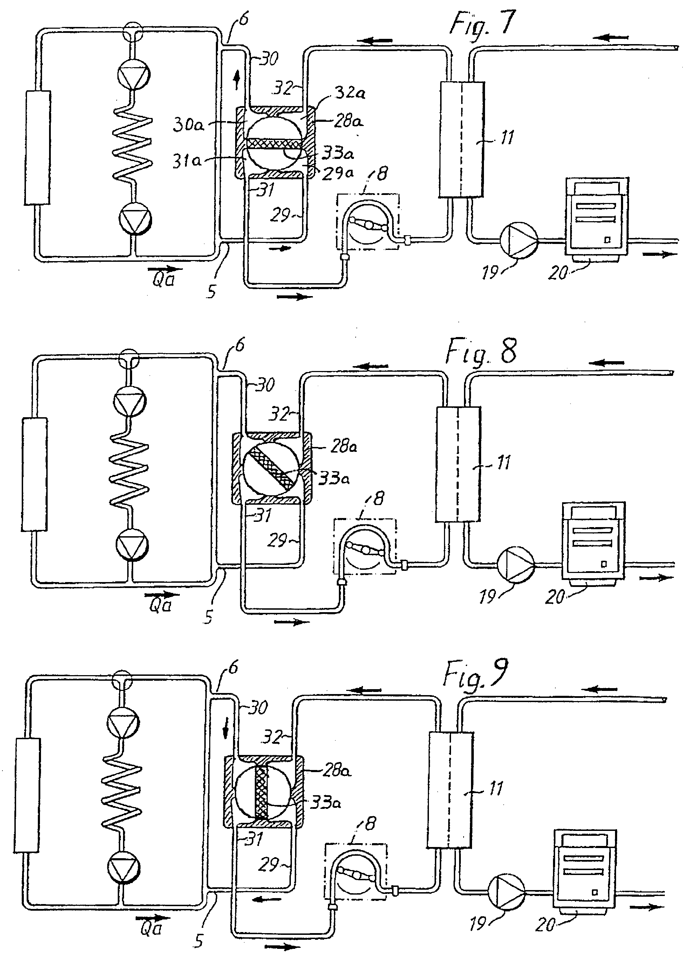

An alternative design of the valve arrangement is shown in Figs 7, 8 and 9.. In the embodiment of Fig. 7, the arterial line 29 is connected to an enlarged opening 29a and the venous outlet line 30 is connected to an enlarged opening 30a, the openings being arranged in the valve housing 28a diametrically opposite to each other. Two enlarged openings 31a and 32a are arranged in the valve housing 28a diametrically opposite each other and displaced 90° in relation to enlarged openings 29a and 30a. The pivotable valve member 33a is normally arranged as shown in Fig.. 7 and forms a partition dividing the valve chamber in two semi-circular portions. The valve member has a width, which is smaller than the peripheral dimension of the enlarged openings.. The valve member is pivotable 90° to a reverse position, shown in Fig. 9, in which the blood flows through the arterial and venous needles are reversed.

During its movement from the normal to the reversed position, the valve member 33a passes through an idle position shown in Fig.. 8, in which all four enlarged openings are interconnected, because the width of the valve member is smaller than the peripheral dimension of the enlarged openings.. By this idle position, harm to blood cells may be avoided. Such harm may be caused by high shear stresses which may occur if the inlet line 31 to the blood pump or the outlet line 32 from the dialyzer are completely occluded. By means of the idle position, another advantage is obtained, that the blood needles are not exposed to rapid change of flows, which in some instances even may result in dislocation of the needles. When the valve member is moved from the normal position to the idle position, the flow through the needles change from the normal flow of, for example, 250 ml/min to essentially zero flow. The valve member may be placed in the idle position for some seconds Then, the valve member is moved to the reversed position, and the flows through the needles is changed from essentially zero flow to -250 ml/min. In this way, a more gentle switch between normal and reversed flows may be obtained.

It is noted, that the positions of the openings and the valve member may be different so that the pivotal movement may be less than or more than 90°. Moreover, the openings need not be arranged diametrically in order to achieve the desired operation. Furthermore, the dimensions of the enlarged openings in relation to the tubes and lines are not in scale, but the diameter of the enlarged openings is rather of the same dimension as the tube inner diameter, as appears more clearly below.

It is noted that the valve is constructed to have as few dead end portions as possible, in which the blood may stand still and coagulate. From the drawing, it is appreciated that no portion of the valve has a dead end construction in any position of the valve body..

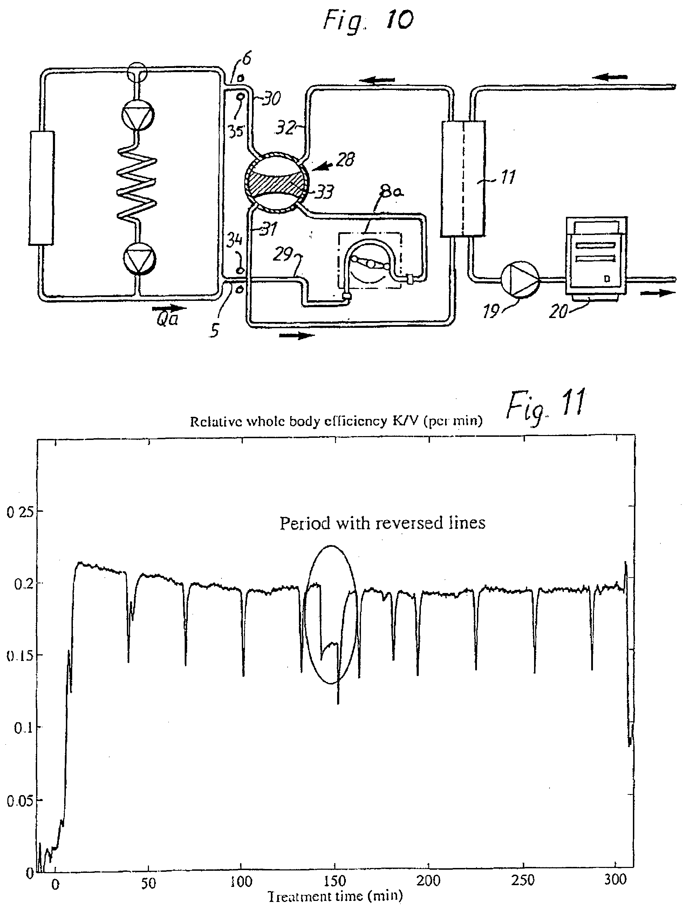

Furthermore, another schematic diagram incorporating a valve is shown in Fig. 10. Fig. 10 differs from Fig. 5 inly in the placement of the pump 8a, which in the embodiment according to Fig. 10 is placed between the arterial needle 5 and the valve 28. In this manner, the pressure across the valve body 33 is less compared to the embodiment according to Fig. 5. The operation is somewhat different. The blood pump is stopped, and the valve is put in the reversed position. Finally, the pump is started and pumping the blood in the opposite direction by reversing the rotational direction of the pump.

In order to ascertain that no air is introduced into the patient in either position of the valve, it may be advantageous to add an air detector 34 and 35 immediately before each of the arterial and venous needle, or at least before the arterial needle. The air detectors trigger an alarm should they measure air bubbles in the blood given back to the blood vessel. Normally, the air detector in the drip chamber is sufficient for this purpose..

The detailed construction of a valve intended to be used in the present invention, is disclosed in Figs. 1.2, 13 and 14. The valve comprises a valve housing 36 comprising two inlet connectors and two outlet connectors. All four connectors open into cylindrical valve chamber 41, the four openings being displaced 90° in relation to each other

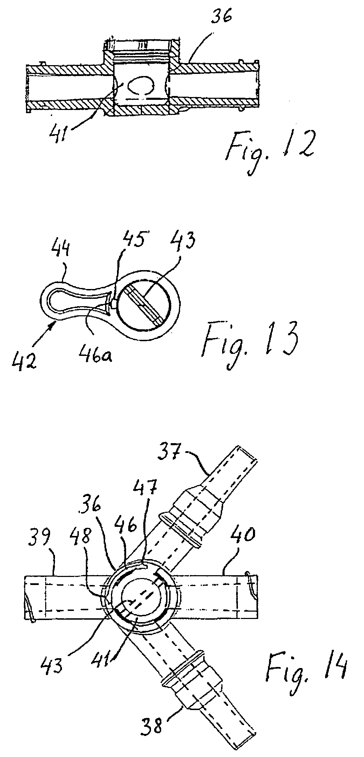

As shown in Fig. 14, the valve comprises a blood inlet connector 37 connected to the arterial needle 5 and a blood outlet connector 38 connected to the venous needle 6. The connector portions are arranged as male Luer connectors to be connected to flexible tubes ending with a female Luer connector.

Furthermore, the valve comprises a circuit outlet connector 39 connected to the blood pump 8 and a circuit inlet connector 40 connected to the dialyzer outlet. The connector portions 39 and 40 are arranged as female Luer connectors to mate with male Luer connectors of the circuit.

As appears from Fig. 12, the cylindrical valve chamber 41. is closed at the bottom. From the top, a valve member 42 may be introduced into the cylindrical valve chamber The valve member 42 comprises a valve partition 43 as appears from Fig. 13.

The valve member also comprises an operating wing 44, by means of which the valve member may be pivoted 90° between a normal position, in which the valve partition 43 is situated as shown by dotted lines in Fig. 14, and a reversed position. The pivotal movement is limited by a shoulder 45 of the valve member 42, which cooperates with a groove 46 in the valve housing. The shoulder 45 is provided with a protrusion 46a which cooperates with two recesses 47 and 48 in the normal position and reverse position, respectively, to maintain the valve member in either position. The groove 46 may be provided with a third recess (not shown in the drawing) in order to define said idle position. Such a third recess is positioned in the middle between the two recesses 47 and 48.

The valve member and housing are provided with suitable sealings to ensure safe operation. The operation of the valve is evident from the above description.

By studying the theoretical dialysate urea concentrations resulting from a given dialyzer clearance K, a given access blood flow Qa and a given blood urea concentration Cs in the systemic venous blood returning from the body, it is found that the effective urea clearance Keff of the dialyzer, taking the cardiopulmonary recirculation into account, is needed for the calculation of access flow. The effective clearance can be measured, for example as described in EP 658 352.

Alternatively, the effective clearance can be calculated from simultaneous systemic venous blood Cs and dialysate Cd measurements of urea concentrations, such as by blood samples.

The systemic blood urea concentration Cs may be measured by the so called stop flow - slow flow technique, where the blood flow is substantially stopped for a couple of minutes to allow the cardiopulmonary recirculation to equalize. Thereafter, the pump is run slowly to fill the arterial line with fresh blood before taking the blood sample. The urea concentration in the so obtained blood sample is equal to the urea concentration Cs in the systemic venous blood returning from the body to the heart.

Alternatively to taking a blood sample, the dialysis fluid flow at the other side of the membrane is stopped and the slowly flowing blood is allowed to equalize with the dialysate at the other side of the membrane, whereupon the urea concentration of the dialysate is measured to obtain the systemic venous blood urea concentration Cs.

A further method to obtain effective clearance is described in WO 9929355. According to the invention described in WO 9929355, the systemic blood concentration Cs is measured before or at the initiation of the treatment, for example by stop flow - slow flow technique with blood sample or equilisation as described above. After obtaining valid dialysate urea concentration vales Cd from a urea monitor connected to the dialysator outlet line, the initial dialysate urea concentration $C_{dinit}$ at the start of the treatment is extrapolated by the dialysate urea curve obtained.

A still further method of obtaining systemic blood urea concentration Cs is to calculate the urea mass $M_{wh}$ in the whole body and extrapolate the urea mass to the start of the treatment. By dividing the whole body urea mass $M_{wh}$ with the distribution volume V, the systemic blood urea concentration Cs at the start of the treatment is obtained.

By dividing the dialysate urea concentration Cd with the systemic blood urea concentration Cs and multiplicating with the dialysate flow rate Qd, the effective clearance Keff is obtained. It is advantageous to measure the effective clearance Keff at the initiation of the treatment.

Furthermore, in the method of the invention, the blood flows in the arterial and venous needles are reversed. The dialysate urea concentrations in the two cases with normal position of the needles and with reverse position of the needles may be calculated as follows, with reference to Figs. 3 and 4.

The blood urea concentration Cs in the venous blood returning from the body is assumed unchanged when the lines are reversed, and the dialyzer clearance K is also assumed unchanged. For simplicity ultrafiltration is assumed to be zero, but it is also possible to handle a nonzero UF.

The following notations are used:

Qco - Cardiac Output
Qa - Access flow
Qb - Blood flow in extracorporeal circuit
Qd - Dialysate flow
K - Dialyzer clearance
Keff - Effective dialyzer clearance
Cs - Blood urea concentration in systemic venous blood returning from the body
Ca - Blood urea concentration in the access
Cb - Blood urea concentration at the dialyzer inlet
Cd - Dialysate urea concentration

The definition of clearance is:

$$K = (\text{removed urea}) / Cb = Qd * Cd / Cb \tag{1}$$

Consider first the case in which Qa > Qb and the needles are in the normal position. In this case Cb = Ca.

Removal from blood must equal appearance in the dialysate so that

$$K * Ca = Qd * Cd \tag{2}$$

A mass balance for urea at the point V, see Fig. 3, when mixing the venous return blood with the blood from the access gives:

$$Ca * Qco = Cs * (Qco - Qa) + Ca * (Qa - K) \tag{3}$$

Thus, we obtain a relation between Ca and Cs..

By combining equations 2 and 3 we obtain:

$$Cd = (K/Qd) * Cs / [1 + K/(Qco -Qa)] \qquad (4)$$

The definition of effective clearance Keff implies that Cs should be used in the denominator instead of Cb as normally used in dialyzer clearance, which means that

$$Keff = K * (Cb / Cs) = K / [1 + K/(Qco -Qa)] \qquad (5)$$

If we now turn to the case with reversed lines, see Fig. 4, we still have that what is removed from the blood must enter the dialysate, so that in this case

$$K * Cb = Qd * Cd \qquad (6)$$

The flow in the fistula between the needles will be Qa + Qb and we can calculate the blood urea concentration at the dialyzer inlet from a urea mass balance at the point P where the dialyzed blood enters the access again

$$Cb * (Qb - K) + Ca * Qa = Cb * (Qb + Qa) \qquad (7)$$

We also have the mass balance at the point Q where the venous return blood meets the dialyzed blood in the access return flow:

$$Ca * Qco = Cs * (Qco - Qa) + Cb * Qa \qquad (8)$$

By eliminating Ca and Cb we get

$$Cd = (K/Qd) * Cs / [1 + (Qco/Qa) * K / (Qco - Qa)] \qquad (9)$$

Since Cs, K and Qd in the two cases are unchanged, it is possible to obtain the ratio of dialysate urea concentrations:

$$Cd\ norm / Cd\ rev = 1 + (K/Qa) / [1 + K/(Qco-Qa)] =$$
$$= 1 + Keff/Qa \qquad (10)$$

In practice, the two dialysate urea concentrations are probably best found by a curve fit to the dialysate urea curves before and after the switch of lines, with an extrapolation to the time of switching from the respective side, see Fig 6, which shows the urea concentration Cd of the dialysate during a normal hemodialysis treatment.
During a time.period of about 10 minutes, marked with a ring in Fig. 6, the arterial and venous needles are reversed. After a initial time period for allowing the urea monitor to measure accurately, the urea concentration with reversed lines is appr. 0.8 times the original urea concentration, which means that Cdnorm / Cdrev = 1.25. Thus, if Keff is 200 ml/min, as measured with the needles in the normal position or estimated as described above, the access flow is 800 ml/min.

The effective clearance may also be obtained as a rough estimate from blood and dialyzer flows and dialyzer characteristics, e.g. from the dialyzer date sheet.

In the present specification, there is used three different clearances, namely dialyzer clearance, effective clearance and whole body clearance. If dialyzer clearance is 250 ml/min for a certain blood flow rate and dialysate flow rate, the effective clearance is normally 5 to 10% lower, such as 230 ml/min. The whole body clearance is still 5 to 15% lower, such as 200 ml/min. The dialyzer clearance is the clearance as measured directly on the dialyzer. The effective clearance is the clearance also taking into account the cardio-pumonary recirculation. Finally, the whole body clearance is the effective clearance further taking into account other membranes in the body restricting the flow of urea from any part of the body to the dialyasate. The concept of whole body clearance is described in WO 9855166.

The effective clearance used in the formula may also be obtained from a measurement according to the method described in EP 658 352 mentioned above, with the needles in the normal position. This will give a measure of the effective plasma water urea clearance, which then has to be converted to whole blood clearance. The method of EP 658 352 essentially comprises that the conductivity of the dialysis fluid upstream of the dialyzer is increased by for example 10% and then returned to the original value. The result at the outlet side of the dialyzer is measured and reults in a measure of the effective clearance Keff of the dialyzer.

Alternatively, the effective clearance may be calculated according to equation Keff = Qd * Cd / Cs. The systemic venous urea concentration may be measured at the same time as the dialysate urea concentration Cd, or by the methods described above.

Another method would be to use the value of total body urea mass Murea obtained by the method according to WO 9855166, mentioned above. By obtaining the urea distribution volume V by Watson's formula or any other method, the venous urea concentration would be approximately:

$$Cs = Murea / V \qquad\qquad (11)$$

In the method of WO 9855166, the relative whole body efficiency of the dialyzing process $K_{wb}/V$ is obtained. Note, that whole body clearance is used, as indicated by the subscript wb. According to said WO 9855166, urea concentration is proportional to the relative whole body efficiency according to the formula:

$$K_{wb}/V = (Q_d \cdot c_d) / m \qquad\qquad (12)$$

Thus, if ($K_{wb}/V$) is used instead of Cd in the above equation (10), a similar result is obtained, if it is presumed that m is constant, i.e the measurement must be extrapolated to the same time instance:

$$(K_{wb}/V) \ norm / (K_{wb}/V) \ rev = 1 + Keff/Qa \qquad\qquad (13)$$

As is mentioned in said WO 9855166, it is possible to calculate the relative whole body efficiency only from dialysate urea measurement. Since we are interested only in the ratio in the normal and reversed position, we do not need to calculate the actual $K_{wh}$.

Fig. 11 shows a plot of the relative whole body efficiency K/V (min$^{-1}$). The period with reversed lines is shown inside a circle. In all other respects, the same discussion applies as is given above.

The calculations above assume that the extracorporeal blood flow rate Qb does not exceed the access flow rate Qa. If this is the case there will be access recirculation and the flow in the access will be reversed when the needles are in the normal position. The calculation of dialysate urea concentration is unchanged for the needles in reversed positi.on, but has to be modified for the needles in normal position. Calculations corresponding to those above show that the ratio above between dialysate urea concentrations for normal and reversed needle positions will be:

$$Cd \ norm / Cd \ rev = 1 + Keff / Qb \qquad\qquad (14)$$

where Keff is the effective clearance with the effect of recirculation included, that is with the needles in the normal position.

The only difference is that the calculation will now give the extracorporeal blood flow Qb instead of the access flow.. This blood flow is known, so in practice this means that when the result is an access flow rate Qa close to the blood flow rate Qb, recirculation should be suspected, and this always means that the access has to be improved.

Keff/Qb is a figure lower than one, normally for example 0.6 - 0.9. Keff/ Qa should be considerably lower, for example 0.1 - 0.4. Thus, when Cd norm/Cd rev approaches or is lower than a predetermined number, such as 1.2 or 1.5, further calculations should be done for determining if access recirculation is present

A simple procedure is to decrease the blood flow Qb somewhat. If the dialysate concentration then decreases, this means that there is no access or fistula recirculation at least at the lower blood flow.

The above calculations can also be made for the situation where ultrafiltration is present. However, it is a simple measure to reduce the ultrafiltration to zero during the measurement interval. Moreover, the error induced by ultrafiltration is small and may be neglected..

The measurement should be performed during a time interval, which is considerably larger than 30 seconds so that cardio-pulmonary recirculation has been developed. The measurement time for obtaining valid results may be 5 minutes with the needles reversed, while measurements with the needles in correct position may be done in 5 minutes or continuously during the treatment.

The method is also applicable to the methods of treatment comprising infusion of a dialysis solution to the blood before or after the dialyzer, called hemofiltration and hemodiafiltration. The result is the same as given above.

If the access is a venous catheter, there is no cardio-pulmonary recirculation and the calculations becomes simpler The result is the same, except that the effective clearance Keff is replaced by the dialyzer clearance K, since the systemic venous urea concentration Cs becomes the same as the dialyzer inlet urea concentration Cb.

It should be noted that all flow rates, clearances and urea concentrations in the calculations relate to whole blood. Approximately 93% of plasma is water, depending on the protein concentration, and about 72% of erythrocytes is water Depending on the hematocrit value, the blood water volume is 10 - 13 % lower than the volume of whole blood, see for example Handbook of Dialysis, Second Edition, John T. Daugirdas and Todd. S Ing, 1994, page 18.

The effective urea clearance obtained according to EP 658 352 relates to blood water, and must therefore be increased by 10 - 13 % before being used in the present formulas. Blood urea concentration values obtained from a laboratory relate in general to plasma, and must therefore be decreased by about 7% in order to relate to whole blood

Alternatively, all urea concentrations, flow rates and clearances may be used as relating to blood water.. The effective clearance is then used unchanged, but the calculated access flow will relate to blood water, and has to be increased by 10 - 13 % to relate to whole blood.

The invention has been described above with reference to use in the human body and using urea as a marker for measuring access flow However, any other substance present in blood and which can be measured at the dialysate side of the dialyzer may be used according to the invention, such as creatinine, vitamin B12, beta-two-microglobuline, NaCl or any combination of ions. Another alternative is to measure conductivity.

It is also possible to measure a property proportional to the concentration, since it is the ratio that is involved in the equations. Thus, urea concentration may be measured by measuring conductivity differences after passing the urea containing fluid through a urease column, and such conductivity difference can be used directly in place of the concentration values in the equations.

Other indirect methods of measuring any of the above-mentioned substances concentrations may be used as long as the measurements are made at the dialysate side of the dialyzer. Another alternative is to measure the blood urea concentrations, by any known method, either before or after the dialyzer, since these concentrations axe proportional to the concentrations in the formulas.

The invention has been described above with reference to use in the human body. However, the invention can be used in any tube system where a fluid is passed and a portion thereof is taken out for dialysis, such as in beer or wine production.

## Claims

1. An apparatus for detecting access recirculation comprising: means for removing a first fluid flow at a first fluid flow rate from a fluid flow access at a removal position to an external flow circuit comprising a dialyzer having a dialysis fluid inlet and a dialysis fluid outlet and having a semipermeable membrane; means for passing said first fluid flow along said membrane at one side thereof and means for emitting dialysis fluid from the other side thereof; means for returning said first fluid flow from said external flow circuit to said access at a return position downstream of said removal position; means for measuring a first variable which is essentially proportional to a concentration of a substance in said dialysis fluid emitted from the dialyzer; means for decreasing the first fluid flow rate; means for monitoring the concentration of said substance in the dialysis fluid emitted from the dialyzer; **characterised by** means for detecting a possible access recirculation that is adapted to detect possible access recirculation by correlating a change in said concentration to said change of the first fluid flow rate such that no access recirculation is detected if the concentration decreases following the decrease in first fluid rate.

2. The apparatus of claim 1, wherein said first fluid flow is blood flow.

3. The apparatus of claim 2, comprising means for decreasing the blood flow rate and means for monitoring a corresponding decrease in the urea concentration, whereby the absence of such a decrease being indicative of fistula recirculation.

4. The apparatus of claim 2, wherein said substance is selected from the group consisting of: urea, creatinine, vitamin B12, beta-two-microglobuline and glucose.

5. The apparatus of claim 2, wherein said substance is an ion selected from the group consisting of: Na+, Cl-, K+, Mg++, Ca++, HCO3-, acetate ion, or any combination thereof; and said concentration is measured as the concentration difference between the outlet and the inlet of the dialyzer.

6. The apparatus of claim 2, wherein said concentration of said substance is measured as the conductivity difference between the dialysis fluid at the outlet of the dialyzer and the dialysis fluid at the inlet of the dialyzer.

7. A method of detecting access recirculation that may develop in an access if a first fluid flow is removed at a first fluid flow rate from said access at a removal position to an external flow circuit comprising a dialyzer and is returned from said external flow circuit to said access at a return position downstream of said removal position; said dialyzer having a dialysis fluid inlet and a dialysis fluid outlet and having a semipermeable membrane so that said first fluid flow can pass along said membrane at one side thereof and a dialysis fluid can pass along said membrane at the other side thereof and be emitted from the dialysis fluid outlet of said dialyzer; said method comprising:

   monitoring the concentration of a substance in the dialysis fluid emitted from the dialyzer during a time period in which the first fluid flow rate has been decreased; and
   detecting a possible access recirculation by correlating a change in said monitored concentration to said known change of the first fluid flow rate such that no access recirculation is detected if the concentration decreases following the decrease in first fluid flow rate.

8. The method of claim 7, wherein said first fluid flow is blood flow and the substance is urea and wherein the step of detecting a possible access recirculation detects access recirculation by detecting an absence of a decrease in the urea concentration when the blood flow has decreased.

9. The method of claim 7, wherein said substance is selected from the group consisting of: urea, creatinine, vitamin B12, beta-two-microglobuline and glucose.

10. The method of claim 7, wherein said substance is an ion selected from the group consisting of: Na+, Cl-, K+, Mg++, Ca++, HCO3-, acetate ion, or any combination thereof; and said concentration is measured as the concentration difference between the outlet and the inlet of the dialyzer.

11. The method of claim 7, wherein said concentration of said substance is measured as the conductivity difference between the dialysis fluid at the outlet of the dialyzer and the dialysis fluid at the inlet of the dialyzer.

**Patentansprüche**

1. Vorrichtung zum Nachweisen von Zugangsrezirkulation, umfassend: Mittel zum Entnehmen eines ersten Fluidstroms mit einer ersten Fluidstromrate aus einem Fluidstromzugang an einer Entnahmeposition zu einem externen Strömungskreis, der ein Dialysegerät mit einem Dialysefluideinlass und einem Dialysefluidauslass und einer semipermeablen Membran umfasst; Mittel zum Leiten des ersten Fluidstroms entlang der Membran an einer Seite davon und Mittel zum Abgeben von Dialysefluid von der anderen Seite davon; Mittel zum Rückführen des ersten Fluidstroms von dem externen Strömungskreis zu dem Zugang an eine Rückführposition stromabwärts bezogen auf die Entnahmeposition; Mittel zum Messen einer ersten Variable, die im Wesentlichen proportional zu der Konzentration eines Stoffs in dem von dem Dialysegerät abgegebenen Dialysefluid ist; Mittel zum Verringern der ersten Fluidstromrate; Mittel zum Überwachen der Konzentration des Stoffs in dem von dem Dialysegerät abgegebenen Dialysefluid; **gekennzeichnet durch** Mittel zum Nachweisen einer möglichen Zugangsrezirkulation, das dafür ausgelegt ist, eine mögliche Zugangsrezirkulation durch Korrelieren einer Veränderung der Konzentration mit der Veränderung der ersten Fluidstromrate nachzuweisen, wobei keine Zugangsrezirkulation nachgewiesen wird, wenn die Konzentrationsabnahmen der Abnahme der ersten Fluidrate folgen.

2. Vorrichtung gemäß Anspruch 1, wobei der erste Fluidstrom Blutstrom ist.

3. Vorrichtung gemäß Anspruch 2, umfassend Mittel zum Verringern der Blutstromrate und Mittel zum Überwachen einer entsprechenden Verringerung der Harnstoffkonzentration, wobei Fehlen einer solchen Abnahme für Fistelrezirkulation indikativ ist.

4. Vorrichtung gemäß Anspruch 2, wobei der Stoff ausgewählt ist aus der Gruppe bestehend aus: Harnstoff, Kreatinin, Vitamin $B_{12}$, beta-zwei-Mikroglobulin und Glucose.

5. Vorrichtung gemäß Anspruch 2, wobei der Stoff ein Ion ist, ausgewählt aus der Gruppe bestehend aus: $Na^+$, $Cl^-$, $K^+$, $Mg^{++}$, $Ca^{++}$, $HCO_3^-$, Acetat-Ion und jeder Kombination davon; und die Konzentration als der Konzentrationsunterschied zwischen dem Auslass und dem Einlass des Dialysegeräts gemessen wird.

6. Vorrichtung gemäß Anspruch 2, wobei die Konzentration des Stoffs als der Leitfähigkeitsunterschied zwischen dem Dialysefluid an dem Auslass des Dialysegeräts und dem Dialysefluid an dem Einlass des Dialysegeräts gemessen wird.

7. Verfahren zum Nachweisen von Zugangsrezirkulation, die sich in einem Zugang entwickeln kann, wenn ein erster Fluidstrom mit einer ersten Fluidstromrate aus dem Zugang an einer Entnahmeposition zu einem externen Strömungskreis, der ein Dialysegerät umfasst, entnommen wird und von dem externen Strömungskreis zu einer Rückführposition, die stromabwärts bezogen auf die Entnahmeposition angeordnet ist, zurückgeführt wird;
wobei das Dialysegerät einen Dialysefluideinlass und einen Dialysefluidauslass aufweist und eine semipermeable Membran aufweist, so dass der erste Fluidstrom an einer Seite davon entlang der Membran fließen kann und ein Dialysefluid an der anderen Seite davon entlang der Membran fließen kann und von dem Dialysefluidauslass des Dialysegeräts abgegeben werden kann;
wobei das Verfahren umfasst:

Überwachen der Konzentration eines Stoffs in dem von dem Dialysegerät abgegebenen Dialysefluid während eines Zeitraums, in dem die erste Fluidstromrate gesenkt worden ist; und
Nachweisen einer möglichen Zugangsrezirkulation durch Korrelieren einer Veränderung der überwachten Konzentration mit der bekannten Veränderung der ersten Fluidstromrate, wobei keine Zugangsrezirkulation nachgewiesen wird, wenn die Konzentrationsabnahmen der Abnahme der ersten Fluidrate folgen.

8. Verfahren gemäß Anspruch 7, wobei der erste Fluidstrom Blutstrom ist und der Stoff Harnstoff ist und wobei der Schritt des Nachweisens einer möglichen Zugangsrezirkulation eine Zugangsrezirkulation nachweist, indem Fehlen einer Abnahme der Harnstoffkonzentration, wenn der Blutstrom abgenommen hat, nachgewiesen wird.

9. Verfahren gemäß Anspruch 7, wobei der Stoff ausgewählt ist aus der Gruppe bestehend aus: Harnstoff, Kreatinin, Vitamin $B_{12}$, beta-zwei-Mikroglobulin und Glucose.

10. Verfahren gemäß Anspruch 7, wobei der Stoff ein Ion ist, ausgewählt aus der Gruppe bestehend aus: $Na^+$, $Cl^-$, $K^+$, $Mg^{++}$, $Ca^{++}$, $HCO_3^-$, Acetat-Ion und jeder Kombination davon; und die Konzentration als der Konzentrationsunterschied zwischen dem Auslass und dem Einlass des Dialysegeräts gemessen wird.

11. Verfahren gemäß Anspruch 7, wobei die Konzentration des Stoffs als der Leitfähigkeitsunterschied zwischen dem Dialysefluid an dem Auslass des Dialysegeräts und dem Dialysefluid an dem Einlass des Dialysegeräts gemessen wird.

**Revendications**

1. Appareil de détection de recirculation d'accès comprenant : un moyen pour retirer un premier écoulement de fluide à un premier débit de fluide à partir d'un accès d'écoulement de fluide à une position de retrait vers un circuit d'écoulement externe comprenant un dialyseur ayant une entrée de fluide de dialyse et une sortie de fluide de dialyse et ayant une membrane semi-perméable ; un moyen pour faire passer ledit premier écoulement de fluide le long de ladite membrane au niveau d'un côté de celle-ci et un moyen pour émettre un fluide de dialyse depuis l'autre côté de celle-ci ; un moyen pour retourner ledit premier écoulement de fluide depuis ledit circuit d'écoulement externe vers ledit accès à une position de retour en aval de ladite position de retrait ; un moyen pour mesurer une première variable qui est essentiellement proportionnelle à une concentration d'une substance dans ledit fluide de dialyse émis depuis le dialyseur ; un moyen pour diminuer le premier débit de fluide ; un moyen pour surveiller la concentration de ladite substance dans le fluide de dialyse émis depuis le dialyseur ; **caractérisé par** un moyen pour détecter une recirculation d'accès possible qui est adapté pour détecter une recirculation d'accès possible par corrélation d'un changement de ladite concentration audit changement du premier débit de fluide de sorte qu'aucune recirculation d'accès ne soit détectée si la concentration diminue après la diminution du premier débit de fluide.

2. Appareil de la revendication 1, dans lequel ledit premier écoulement de fluide est une circulation sanguine.

3. Appareil de la revendication 2, comprenant un moyen pour diminuer le débit de sang et un moyen pour surveiller une diminution correspondante de la concentration d'urée, de sorte que l'absence d'une telle diminution soit indicatrice de recirculation de fistule.

4. Appareil de la revendication 2, dans lequel ladite substance est choisie dans le groupe constitué de : l'urée, la créatinine, la vitamine B12, la bêta-deux-microglobuline et le glucose.

5. Appareil de la revendication 2, dans lequel ladite substance est un ion choisi dans le groupe constitué de : Na+, Cl-, K+, Mg++, Ca++, HC03-, l'ion, acétate, ou une combinaison quelconque de ceux-ci ; et ladite concentration est mesurée comme étant la différence de concentration entre la sortie et l'entrée du dialyseur.

6. Appareil de la revendication 2, dans lequel ladite concentration de ladite substance est mesurée comme étant la différence de conductivité entre le fluide de dialyse à la sortie du dialyseur et le fluide de dialyse à l'entrée du dialyseur.

7. Procédé de détection d'une recirculation d'accès qui peut se développer dans un accès si un premier écoulement de fluide est retiré à un premier débit de fluide dudit accès à une position de retrait vers un circuit d'écoulement externe comprenant un dialyseur et est retourné depuis ledit circuit d'écoulement externe vers ledit accès à une position de retour en aval de ladite position de retrait ;
ledit dialyseur ayant une entrée de fluide de dialyse et une sortie de fluide de dialyse et ayant une membrane semi-perméable de sorte que ledit premier écoulement de fluide puisse passer le long de ladite membrane au niveau d'un côté de celle-ci et un fluide de dialyse puisse passer le long de ladite membrane à l'autre côté de celle-ci et soit émise à partir de la sortie de fluide de dialyse dudit dialyseur ;
ledit procédé comprenant :

la surveillance de la concentration d'une substance dans le fluide de dialyse émis depuis le dialyseur pendant une période dans laquelle le premier débit de fluide a été diminué ; et
la détection d'une recirculation d'accès possible par corrélation d'un changement de ladite concentration surveillée audit changement connu du premier débit de fluide de sorte qu'aucune recirculation d'accès ne soit détectée si la concentration diminue après la diminution du premier débit de fluide.

8. Procédé de la revendication 7, dans lequel ledit premier écoulement de fluide est une circulation sanguine et la substance est l'urée et dans lequel l'étape de détection d'une recirculation d'accès par détection d'une absence d'une diminution de la concentration d'urée lorsque l'écoulement de sang a diminué.

9. Procédé de la revendication 7, dans lequel ladite substance est choisie dans le groupe constitué de : l'urée, la créatinine, la vitamine B12, la bêta-deux-microglobuline et le glucose.

10. Procédé de la revendication 7, dans lequel ladite substance est un ion choisi dans le groupe constitué de : Na+, Cl-, K+, Mg++, Ca++, HC03-, l'ion acétate, ou une combinaison quelconque de ceux-ci ; et ladite concentration est mesurée comme étant la différence de concentration entre la sortie et l'entrée du dialyseur.

**11.** Procédé de la revendication 7, dans lequel ladite concentration de ladite substance est mesurée comme étant la différence de conductivité entre le fluide de dialyse à la sortie du dialyseur et le fluide de dialyse à l'entrée du dialyseur.

Fig.1

Fig.2

**Fig. 3**

**Fig. 4**

## Fig. 5

## Fig. 6

Fig. 7

Fig. 8

Fig. 9

## Fig. 10

## Fig. 11

Relative whole body efficiency K/V (per min)

Period with reversed lines

Treatment time (min)

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5685989 A
- US 5595182 A
- US 5453576 A
- US 5510716 A
- US 5510717 A
- US 5312550 A
- US 5605630 A
- US 5894011 A
- DE 19541783 C1
- DE 19702441 C1
- WO 9855166 A
- EP 658352 A
- WO 9929355 A

**Non-patent literature cited in the description**

- **JOHN T. DAUGIRDAS ; TODD. S ING.** Handbook of Dialysis. 1994, 18